# EUROPEAN PATENT APPLICATION

(11) **EP 4 368 275 A1**
(43) Date of publication of application: **15.05.2024**
(21) Application number: 22216984.9
(22) Date of filing: 28.12.2022
(51) Int. Cl.: B01D 53/047, A61M 16/00, C01B 13/00

(54) **SYSTEM AND METHOD FOR GENERATING A HIGH PURITY PRODUCT GAS**

(30) Priority: 14.11.2022 KR 20220151611
(71) Applicant: Sunbio2 Co.,Ltd., Yongin-si, Cheoin-Gu, Gyeonggi-Do 17159 (KR)
(72) Inventor: Jin, Bong Yeon, 17159 Cheoin-gu, Gyeonggi-do (KR); Choi, Seung-Keun, 17159 Cheoin-gu, Gyeonggi-do (KR); Jeon, Geum-Bai, 17159 Cheoin-gu, Gyeonggi-do (KR)
(74) Representative: Gulde & Partner

(57) **Abstract**

An objective of the present disclosure is to provide a high-purity refinement system and method that can stably obtain a high-purity object gas in comparison to a pressure adsorption method of the related art by securing a process for a dynamic pressure time to improve pressure swing, which is generated in a producing and recycling process of the pressure adsorption method of the related art, and to increase production efficiency.

The high-purity gas refinement system according to the present disclosure includes a first adsorption bed and a second adsorption bed having an adsorbent therein, connected to a discharge line and a collection line at a first side, and connected to a first inflow line and a dynamic pressure line at a second side, in which a dynamic pressure time that is provided when the first adsorption bed and the second adsorption bed are switched is separately provided to an upper portion and a lower portion, and at the lower portion, a supply gas that is supplied through the dynamic pressure lines to the lower portion of the first adsorption bed and the lower portion of the second adsorption bed for a dynamic pressure time before the beds are switched is supplied and dynamically pressurized as a gas the same as an inflow gas that is supplied through the first inflow lines.

## Description

### BACKGROUND OF THE INVENTION

### Field of the Invention

The present disclosure relates to a high-purity refinement system and method and, more particularly, to a high-purity refinement system and method that can obtain a high-purity object gas.

### Description of the Related Art

At present, gas generators are used for various purposes in various fields.

For example, an oxygen generator that is one of gas generators is generally used in most cases to recover from fatigue accumulated in daily life of modern people and to activate cells by supplying oxygen into a closed space at offices or common home.

Further, a medical gas generator, an automotive gas generator, an experiment gas generator, etc. are widely used.

As general gas separation, in a broad meaning, chemical reaction, electrolysis, physical separation, etc. are used. As the physical separation, there are membrane separation that separates a gas using the difference in polarity of substances of the gas and the difference in gas molecule size, and Pressure Swing Adsorption (PSA) that separates a gas using an adsorption/desorption principle of a crystalline solid compound.

PSA, which is a technology of extracting and concentrating an object gas from a gas mixture using pressure swing, is based on the difference in selectivity of components of a gas mixture to an adsorbent.

PSA is a method that is used to separate and refine a gas by separating or removing specific components from a gas mixture, and according to this method, components with a high selectivity (hereafter, referred to as high-adsorbent components) are adsorbed first and components with a low selectivity (hereafter, referred to as a low-adsorbent components) are discharged out of an adsorption bed while a gas mixture passes at high temperature through the adsorption bed filled with a molecular sieve adsorbent.

The pressure in the adsorption bed is decreased to remove the adsorbed components and the adsorption bed is rinsed with some of high-pressure products.

Products can be continuously obtained by repeating this series of processes.

In particular, PSA using two adsorption beds in the related art is a method in which a gas mixture is alternately supplied to the two adsorption bed with a predetermined cycle. A gas mixture flows into the beds until a separable time (in general, around several minutes), and when a bed is saturated with an adsorbent and the separation ability starts to decrease, the bed is replaced with another bed, a gas flows into the another bed. Further, the saturated bed is depressurized from the bottom and the saturation gas is vented.

In particular, in a method of concentrating, refining, and separating a gas that is supplied to a gas refinement apparatus composed of two or more adsorption beds, when continuous production is possible by repeating producing and recycling, a high-purity object gas can be obtained.

That is, in PSA, producing and recycling are alternately performed with a predetermined interval by two adsorption beds, that is, when one adsorption bed finishes producing for a predetermined time, the bed is replaced with another adsorption bed recycled through dynamic pressure time, whereby products can be continuously obtained.

This has been developed as a technology that is generally used in many fields, and at present, it is being developed in priority of energy saving and efficiency, and stability.

However, there is a problem in PSA of the related art that unless the equipment is turned off when products are not used, this is a state in which producing and recycling are repeatedly performed, so energy is consumed equally to the state in which products are used.

Further, this method is focused on only collection of a gas refined at the upper end of adsorption beds, so there is a possibility of damage to the adsorbent in the adsorption bed due to shock of pressure swing that rapidly changes when beds are changed, so there is a problem with stability of the adsorbent.

### (Prior Art Document)

(Patent Document) Korean Patent No. 10-0619290

### SUMMARY OF THE INVENTION

An objective of the present disclosure for solving the problems described above in the related art is to provide a high-purity refinement system and method that can stably obtain a high-purity object gas in comparison to a pressure adsorption method of the related art by securing a process for a dynamic pressure time to improve pressure swing, which is generated in a producing and recycling process of the pressure adsorption method of the related art, and to increase production efficiency.

In order to achieve the objectives, a high-purity gas refinement system according to the present disclosure includes a first adsorption bed and a second adsorption bed having an adsorbent therein, connected to a discharge line and a collection line at a first side, and connected to a first inflow line and a dynamic pressure line at a second side, in which a dynamic pressure time that is provided when the first adsorption bed and the second adsorption bed are switched is separately provided to an upper portion and a lower portion, and at the lower portion, a supply gas that is supplied through the dynamic pressure lines to the lower portion of the first adsorption bed and the lower portion of the second adsorption bed for a dynamic pressure time before the beds are switched is supplied and dynamically pressurized as a gas the same as an inflow gas that is supplied through the first inflow lines.

Further, in the high-purity gas refinement system according to the present disclosure, at the lower portion, a first inflow line through which a supply gas is supplied, the dynamic pressure lines, and a second inflow line connected to the dynamic pressure line are connected in parallel.

Further, in the high-purity gas refinement system according to the present disclosure, a supply gas that is supplied through the first inflow line and a supply gas that is supplied through the second inflow line for dynamic pressure in recycling are simultaneously supplied.

Further, the high-purity gas refinement system according to the present disclosure includes: a pressure sensor and a flow rate sensor in the discharge line; and a control system adjusting a producing time and recycling time of an object gas on the basis of predetermined conditions of the pressure sensor and the flow rate sensor, depending on a use amount of the object gas.

Further, the high-purity gas refinement system according to the present disclosure includes a plurality of first check valve in the first inflow line, in which a gas that is supplied to the dynamic pressure lines when the beds are switched is separately provided to the first adsorption bed and the second adsorption bed through the first check valves.

Further, in the high-purity gas refinement system according to the present disclosure, a plurality of third check valves installed in a supply direction of a supply gas and preventing backflow of supplied gas flow and a second solenoid valve controlling the flow of a supplied gas are included in the dynamic pressure lines.

Further, in the high-purity gas refinement system according to the present disclosure, a plurality of second check valves installed in a collection direction of an object gas and preventing backflow of collected gas flow and a first solenoid valve controlling the flow of a collected gas are included in the collection lines.

Further, in the high-purity gas refinement system according to the present disclosure, a gas for recycling is collected through the collection lines for the dynamic pressure time at the upper portion.

Meanwhile, in order to achieve the objectives, a high-purity gas refinement method according to the present disclosure includes: a step in which a gas is sent inside and pressurized through a first inflow line connected to a first adsorption bed, whereby a high-adsorbent component is adsorbed and an object gas is discharged through a discharge line connected to the first adsorption bed; a step in which a remaining object gas is sent into a second adsorption bed through collection lines connected to the first adsorption bed before the first adsorption bed is saturated with the high-adsorbent component; and a step in which the first adsorption bed is vented and a supply gas is sent inside and pressurized through collection lines connected to the second adsorption bed, in which the steps are periodically switched and repeated in the first adsorption bed and the second adsorption bed, a dynamic pressure time that is provided when the first adsorption bed and the second adsorption bed are switched is separately provided to an upper portion and a lower portion, and at the lower portion, a supply gas that is supplied through the dynamic pressure lines to the lower portion of the first adsorption bed and the lower portion of the second adsorption bed for a dynamic pressure time before the beds are switched is supplied and dynamically pressurized as a gas the same as an inflow gas that is supplied through the first inflow lines.

Further, in the high-purity gas refinement method according to the present disclosure, at the lower portion, a first inflow line through which a supply gas is supplied, the dynamic pressure lines, and a second inflow line connected to the dynamic pressure line are connected in parallel.

Details of other embodiments are included in detailed description of the invention" and the accompanying "drawings".

The advantages and/or features of the present disclosure, and methods of achieving them will be clear by referring to the exemplary embodiments that will be describe hereafter in detail with reference to the accompanying drawings.

However, it should be noted that the present disclosure is not limited to the configuration of each of embodiments to be described hereafter and may be implemented in various ways, and the exemplary embodiments described in the specification are provided to complete the description of the present disclosure and let those skilled in the art completely know the scope of the present disclosure and the present disclosure is defined by claims.

According to the present disclosure, there is an effect that it is possible to stably obtain a high-purity object gas in comparison to a pressure adsorption method of the related art by securing a process for a dynamic pressure time to improve pressure swing, which is generated in a producing and recycling process of the pressure adsorption method of the related art, and to increase production efficiency.

### BRIEF DESCRIPTION OF THE DRAWINGS

The above and other objectives, features and other advantages of the present invention will be more clearly understood from the following detailed description when taken in conjunction with the accompanying drawings, in which:
FIG. 1 is a diagram showing the configuration of a high-purity refinement system according to an embodiment of the present disclosure;
FIG. 2 is a diagram showing the configuration of a high-purity refinement system and a control system according to an embodiment of the present disclosure; and
FIG. 3 is a flowchart showing the entire flow of a high-purity refinement method according to an embodiment of the present disclosure.

### DETAILED DESCRIPTION OF THE INVENTION

Before describing the present disclosure in detail, terms or words used herein should not be construed as being limited to common or dictionary meanings, the concepts of various terms may be appropriately defined to the most optimally describe the invention by the inventor(s), and it should be noted that those terms or words should be construed as meanings and concepts corresponding to the technical spirit of the present disclosure.

That is, it should be noted that the terms used herein are used only to describing preferred embodiments of the present disclosure, not intending to limit the present disclosure in detail, and those terms are terms defined in consideration of various possibilities of the present disclosure.

Further, it should be noted that, in the specification, singular expression may include plural expression unless clearly stated in the sentences, and includes a singular meaning even if it is similarly expressed as a plural number.

It should be noted that when a component is described as "including" another component throughout the specification, the component may further include another component without another component excluded, unless specifically stated otherwise.

Further, it should be noted that when a component is described as "exists in" and "is connected to" another component, the component may be directly connected with another component, may be installed in contact with another component, or may be installed with a predetermined gap. When the component is installed with a gap, there may be a third component or means for fixing and connecting the component to another component, and the third component or means may not be described.

On the other hands, it should be understood that when a component is described as "directly connected" or "indirectly connected" to another component, it should be construed as there is no third component or means.

Similarly, the terms used herein to describe a relationship between elements, that is, "between", "directly between", "adjacent" or "directly adjacent" should be interpreted in the same manner as those described above.

Further, in the specification, it should be noted that terms such as "first side", "second side", "first", and "second", if used, are used to clearly discriminate one components from another component and the meaning of the corresponding component is not limited by the terms.

Further, terms related to positions such as "up", "down", "left", and "right", if used herein, should be construed as indicating relative positions of corresponding components in the corresponding figures and should not be construed as stating absolute positions unless the absolute positions of them are specified.

Further, in the specification, when components are given reference numerals, the same reference numerals are given to same components even if they are shown in different figures, that is, same reference numerals indicate same components throughout the specification.

The size, position, coupling relationship, etc. of components of the present disclosure may be partially exaggerated or reduced in the accompanying drawings for the convenience of description in order to sufficiently and clearly transmit the spirit of the present disclosure, so the proportion or scale may not be precise.

Further, in the following description of the present disclosure, components that are determined to unclearly make the spirit of the present disclosure unclear, for example, well-known technology including the related art may not be described in detail.

Hereafter, embodiments of the present disclosure are described in detail with reference to relevant drawings.

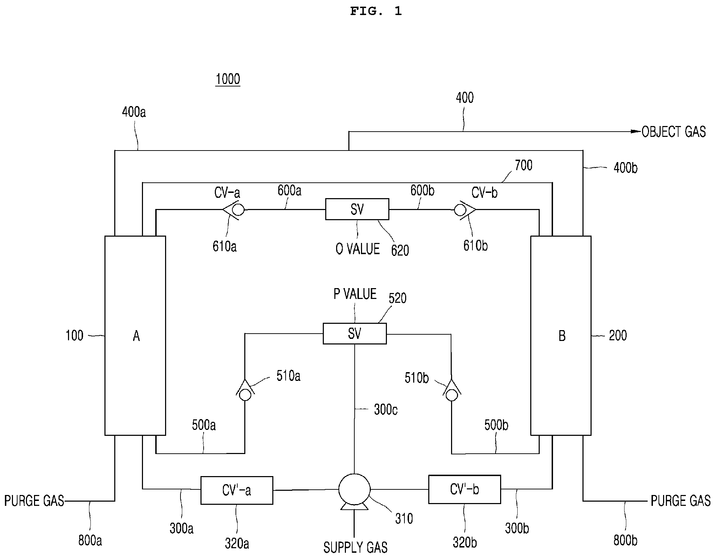

FIG. 1 is a diagram showing the configuration of a high-purity refinement system according to an embodiment of the present disclosure.

Referring to FIG. 1, Pressure Swing Adsorption (PSA) of a high-purity refinement system 1000 according to this embodiment is a method of adsorbing high-adsorbent components and high-purity low-adsorbent components, that is, an object gas by pressurizing and passing a gas mixture, that is, a supply gas through a first adsorption bed 100 and a second adsorption bed 200 that are entirely filled with a molecular sieve that is an adsorbent.

This, as described above, uses selective adsorption to a specific component of an adsorbent due to a pressure difference, the adsorbent depends on high-adsorbent components of a supply gas to be removed or the kind of the object gas but various sieves such as Zeolite Molecular Sieve (ZMS) and Carbon Molecular Sieve (CMS) are used, and in this embodiment, pressure swing adsorption method and apparatus that produce a high-purity oxygen gas using synthetic zeolite such as A-type zeolite (MS5A) ion-exchanged with Ca having selective adsorption to nitrogen or X-type zeolite (CaX) of a faujasite structure are exemplified.

In the PSA according to this embodiment, fundamentally, a supply gas is alternately pressurized, depressurized, adsorbed, and separated through the first adsorption bed 100 and the second adsorption bed 200, whereby an object gas, that is, a high-purity oxygen gas is continuously produced. The adsorption and separation process of the first adsorption bed 100 will be mainly described in detail with reference to FIG. 1, and detailed description adsorption and separation process of the first adsorption bed 100 that are alternately repeated, that is, periodically switched is omitted.

First, in the configuration that supplies and pressurizes a supply gas into the first adsorption bed 100 that has been vented and rinsed, common air is used as the supply gas, but air that has undergone preprocessing such as dehumidifying or filter may be used as the supply gas, depending on the use of the object gas that is the target to be produced.

A supply gas flows inside through a first inflow line 300a and is pressurized up to a predetermined pressure at the lower end of the first adsorption bed 100, and a gas supplies that is used in this case includes a suction pump 310 and an opening/closing device (a valve, not shown) controlling the suction pump 310.

In a configuration that adsorbs high-adsorbent components of the first adsorption bed 100, while a supply gas passes through the first adsorption bed 100 from the lower end to the upper end thereof at a predetermined pressure, an oxygen gas that is a high-adsorbent component is adsorbed by an adsorbent that is a synthetic zeolite molecular sieve in the first adsorption bed 100.

In a configuration that extracts a high-purity object gas from the first adsorption bed 100, an object gas, that is, an oxygen gas is discharged through a discharge line 400a connected to the upper end of the first adsorption bed 100 until the adsorption ability of the adsorbent is exhausted.

In this configuration, when the purity of an object gas is 99%, purity of about 99% that is target purity of the object gas is maintained by closing a valve (not shown) in the discharge line 400a before the adsorption ability of the adsorbent is exhausted, that is, the purity of the object gas drops, and in this case, an oxygen gas not reaching the target purity (99%) but having high purity over 90% exists at the upper portion of the first adsorption bed 100.

A configuration in which the high-purity object gas remaining at the upper portion of the first adsorption bed 100 flows into the second adsorption bed 200 is described.

As described above, the valve (not shown) in the discharge line 400a is closed before the purity of an object gas drops, and an oxygen gas having high purity over 90% and existing at the upper portion of the first adsorption bed 100 is supplied to the upper end of the second adsorption bed 200, which has been recycled through venting and rinsing, through collection lines 600a and 600b.

That is, in the high-purity refinement system 1000 according to the present disclosure, at an upper portion, a gas for recycling is collected through the collection lines 600a and 600b for a dynamic pressure time.

As described above, the method and configuration for injecting a high-purity oxygen gas remaining at the upper portion of the first adsorption bed 100 to the upper end of the second adsorption bed 200 are suitable when a small or large adsorption bed is used.

The collection lines 600a and 600b according to this embodiment are composed of collection lines 600a and 600b connecting gas flow from the upper portion of the first adsorption bed 100 to the upper end of the second adsorption bed 200, a first solenoid valve (SV) 620 installed in the collection lines 600a and 600b and controlling the gas flow, and a plurality of second check valves 610a preventing backflow of the gas flow.

That is, the high-purity refinement system 1000 according to the present disclosure includes a plurality of second check valves 610b installed in a collection direction of an object gas and preventing backflow of gas flow that is collected and a first solenoid valve 620 controlling the flow of collected gas in the collection lines 600a and 600b.

Of course, the system includes also the collection lines 600a and 600b connecting gas flow from the upper portion of the second adsorption bed 200 to the upper end of the first adsorption bed 100, a first solenoid valve (SV) 620 installed in the collection lines 600a and 600b and controlling the gas flow, and a plurality of second check valves 610b preventing backflow of the gas flow.

As described above, the oxygen gas having purity over 90% and existing at the upper portion of the first adsorption bed 100 immediately after a high-purity object gas is discharged from the first adsorption bed 100 by controlling the first solenoid valve 620 flows into the upper end of the second adsorption bed 200 and becomes a portion of the supply gas of the second adsorption bed 200, whereby the oxygen gas becomes later a high-purity object gas through an adsorption process of the second adsorption bed 200.

In this configuration, it is preferable that the gas that flows into the upper end of the second adsorption bed 200 through the collection lines 600a and 600b and then becomes the supply gas of the second adsorption bed 200 accounts for around 20% of the second adsorption bed 200.

That is, in PSA using two adsorption beds in the related art, an object gas not reaching target purity but having purity over 90% and remaining at the upper portion of one adsorption bed that has undergone adsorption and extraction steps is vented or purged to recycle the adsorption bed, so there is a problem that a high-purity object gas is wasted. However, the PAS according to this embodiment repeatedly performs adsorption and extraction steps while receiving the object gas as a supply gas collecting and sending the object gas through another adsorption bed, thereby providing a method that can efficiently obtain a high-purity object gas without wasting energy.

Next, a configuration that sends a supply gas to the upper portion of the second adsorption bed 200 through the collection lines 600a and 600b and pressurizes the supply gas while venting the first adsorption bed 100 is described.

A vent line 800a connected to the lower end of the first adsorption bed 100 is opened and pressure is decreased to separate a nitrogen gas that is a high-adsorbent component saturated in the first adsorption bed 100, whereby the nitrogen gas is vented as a purge gas.

It is preferable that a supply gas is sent and pressurized through dynamic lines 600a and 600b of the second adsorption bed 200, which has been recycled, for a dynamic pressure time at which the internal pressures of the first adsorption bed 100 and the second adsorption bed 200 become the same simultaneously with venting of the first adsorption bed 100.

Thereafter, adsorption and separation processes are repeated in the second adsorption bed 200.

That is, the configuration of adsorbing a nitrogen gas that is a high-adsorbent component of the second adsorption bed 200 and the configuration of extracting a high-purity object gas from the second adsorption bed 200 are sequentially performed, and a portion of an oxygen gas extracted from the upper end of the second adsorption bed 200 through an orifice line 700, which functions as an air channel having a relatively small diameter, is introduced to the upper end of the first adsorption bed 100 that has finished venting and an oxygen gas remaining therein is rinsed almost simultaneously with the configuration of extracting a high-purity object gas from the second adsorption bed 200, whereby the first adsorption bed 100 is completely recycled.

Further, the second adsorption bed 200 undergoes a process of sending a high-purity oxygen gas existing at the upper portion thereof into the upper end of the first adsorption bed 100, which has been recycled, through the collection lines 600a and 600b after a high-purity object gas is extracted from the second adsorption bed 200.

As described above, a pair of first adsorption bed 100 and the second adsorption bed 200 continuously and alternately performs adsorption and separation processes using pressure swing, and particularly, supplies a high-purity oxygen gas remaining at the upper ends thereof after an object gas of the adsorption beds is extracted through the collection lines 600a and 600b, respectively, to the upper end of another adsorption bed that has been recycled, whereby a high-purity object gas can be efficiently obtained with waste of small amount of energy.

Further, since one adsorption bed becomes a high-pressure state at the end of extraction of an object gas and the other one adsorption bed that has finished venting and rinsing steps becomes a relatively low-pressure state, the gas flow through the collection lines is made by controlling the first solenoid valve 620 on the basis of this pressure gradient without a specific additional device such as an air compressor, so there is an advantage that the costs for a facility and operation can be reduced.

Meanwhile, as described above, the gas existing at the upper portion of the first adsorption bed 100 is collected to the upper portion of the second adsorption bed 200, but the gas remaining at the lower portion of the first adsorption bed 100 has purity lower than that of the gas existing at the upper portion.

However, as described above, when only the gas existing at the upper portion of the first adsorption bed 100 is collected to the second adsorption bed 200, productivity decreases.

Further, when producing and recycling of a gas are performed by the configurations described above and when the equipment is not turned off even while products are not used, this is a state in which producing and recycling are repeatedly performed and energy is consumed equally to when products are used. Further, since the system is focused only on collecting a gas refined at the upper portion of an adsorption bed, there is a possibility of damage to the adsorbent in the adsorption bed due to shock of pressure swing that rapidly changes when beds are changed, so there is a problem with stability of the adsorbent.

Accordingly, the high-purity refinement system 1000 according to the present disclosure includes the first adsorption bed 100 and the second adsorption bed 200 that have an adsorbent therein, are connected at first sides to the discharge lines 400a and 400b and the collection lines 600a and 600b, and are connected at second sides to the first inflow lines 300a and 300b and the dynamic pressure lines 500a and 500b, in which a dynamic pressure time that is provided when the first adsorption bed 100 and the second adsorption bed 200 are switched is separately provided to an upper portion and a lower portion.

A configuration in which a gas for recycling is collected at the upper portions of the first adsorption bed 100 and the second adsorption bed 200 for a dynamic pressure time that is provided to an upper portion when beds are switched was described above, and a configuration that is performed at the lower portions of the first adsorption bed 100 and the second adsorption bed 200 for a dynamic pressure time that is provided to a lower portion when beds are switched is described hereafter.

The high-purity refinement system 1000 according to the present disclosure includes, at a lower portion, the first inflow lines 300a and 300b, a second inflow line 300c, a suction pump 310, dynamic pressure lines 500a and 500b, a plurality of third check valves 510a and 510b, and a second solenoid valve 520.

At the lower portion of the high-purity refinement system 1000 according to the present disclosure, the supply gas that is supplied through the dynamic pressure lines 500a and 500b to the lower portions of the first adsorption bed 100 and the second adsorption bed 200 for a dynamic pressure time before beds are switched is supplied and dynamically pressurized as a gas the same as the inflow gas that is supplied through the first inflow lines 300a and 300b.

Further, at the lower portion of the high-purity refinement system 1000 according to the present disclosure, the first inflow lines 300a and 300b through which a supply gas is supplied, the dynamic pressure lines 500a and 500b, and a second inflow line 300c connected to the dynamic pressure lines 500a and 500b are connected in parallel.

In this case, for production, the supply gas that is supplied through the first inflow lines 300a and 300b and the supply gas that is supplied through the second inflow line 300c for dynamic pressure in recycling may be simultaneously supplied.

Accordingly, a gas that is the same as the gas that is supplied through the first inflow lines 300a and 300b is supplied through the second inflow line 300c to the lower portion of the first adsorption bed 100 and the lower portion of the second adsorption bed 200. Further, the gas is supplied to the lower portion of the first adsorption bed 100 and the lower portion of the second adsorption bed 200 through the dynamic pressure lines 500a and 500b, there is an effect that it is possible to prevent a possibility of damage to the adsorbent in the adsorption beds due to shock of pressure swing that rapidly changes when beds are switched.

Further, the high-purity refinement system 1000 according to the present disclosure includes a pressure sensor and a flow rate sensor in the discharge lines 400a, 400b, and 400, and may include a control system 2000 that communicates with the pressure sensor and the flow rate sensor.

The pressure sensor senses the pressure of the first adsorption bed 100 and the second adsorption bed 200 and the flow rate sensor senses the amount of gas of the first adsorption bed 100 and the second adsorption bed 200.

Further, the control system 2000 adjusts the producing time and recycling time of an object gas on the basis of predetermined conditions of the pressure sensor and the flow rate sensor, depending on the use amount of the object gas.

That is, it is possible to be always supplied with a predetermined amount of a high-purity refined gas by installing the pressure sensor and the flow rate sensor in the discharge lines 400a, 400b, and 400 and changing the producing time and the recycling time through the control system 2000, depending on the use amount of the object gas that is discharged. Further, since a high-purity gas is maintained even through the object gas is used in small amount or is not used, so stability of purity is secured. Further, it is possible to reduce energy that is consumed by the high-purity refinement system 1000 by controlling the recycling time and the producing time, depending on the use amount. Further, a gas that is the same as the collection gas is supplied to the adsorption beds when the beds are switched, so the pressure swing in the adsorption beds is small, whereby stability of the adsorbent can be achieved.

Meanwhile, the high-purity refinement system 1000 according to the present disclosure includes a plurality of first check valves 320a and 320b in the first inflow lines 300a and 300b.

The supply gas that is supplied to the dynamic pressure lines 500a and 500b when beds are switched is separately provided to the first adsorption bed 100 and the second adsorption bed 200 through the plurality of first check valves 320a and 320b.

Further, the high-purity refinement system 1000 according to the present disclosure includes a plurality of third check valves 510a and 510b installed in a supply direction of a supply gas and preventing backflow of gas flow and a second solenoid valve 520 controlling the flow of a supplied gas in the dynamic pressure lines 500a and 500b.

A P value can be obtained by the second solenoid valve 520.

Further, an O value can be obtained by the first solenoid valve 620 described above.

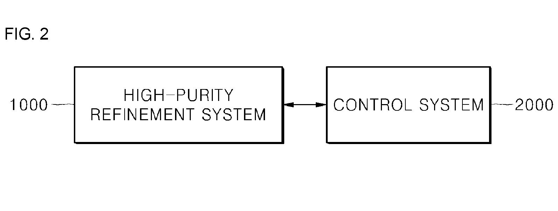

FIG. 2 is a diagram showing the configuration of a high-purity refining system and a control system according to an embodiment of the present disclosure.

Referring to FIG. 2, the high-purity refinement system 1000 according to an embodiment of the present disclosure adjusts a producing time and a recycling time of an object gas through the control system 2000 on the basis of predetermined conditions of the pressure sensor and the flow rate sensor described above on the amount of a use amount of an object gas.

That is, it is possible to be always supplied with a predetermined amount of a high-purity refined gas by installing the pressure sensor and the flow rate sensor in the discharge lines 400a, 400b, and 400 and changing the producing time and the recycling time through the control system 2000, depending on the use amount of the object gas. Further, since a high-purity gas is maintained even through the object gas is used in small amount or is not used, so stability of purity is secured. Further, it is possible to reduce energy that is consumed by the high-purity refinement system 1000 by controlling the recycling time and the producing time, depending on the use amount. Further, a gas that is the same as the collection gas is supplied to the adsorption beds when the beds are switched, so the pressure swing in the adsorption beds is small, whereby stability of the adsorbent can be achieved.

Meanwhile, the pressure sensor and the flow rate sensor of the high-purity refinement system 1000 wirelessly communicate with the control system 2000.

Such wireless communication may be performed by wireless communication modules that support various wireless communication types such as global System for Mobile (GSM) communication, Code Division Multiple Access (CDMA), Wideband Code Division Multiple Access (WCDMA), a universal mobile telecommunications system (UMTS) Time Division Multiple Access (TDMA), Long Term Evolution (LTE), etc. besides a Wifi module and a wireless broadband module.

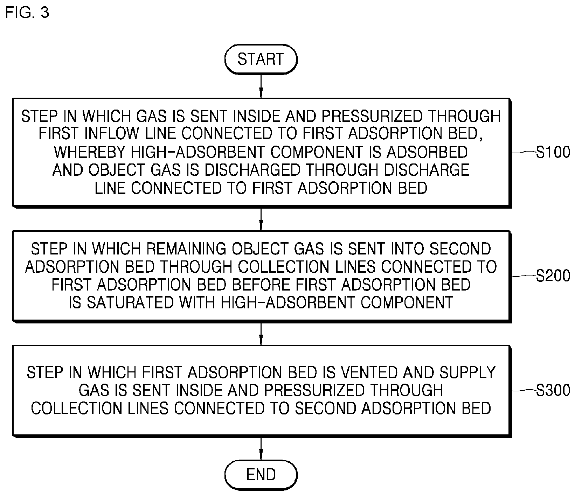

FIG. 3 is a flowchart showing the entire flow of a high-purity refinement method according to an embodiment of the present disclosure.

In a high-purity refinement method according to an embodiment of the present disclosure, the same components as the components of the high-purity refinement system 1000 described above are not described in detail.

Referring to FIG. 3, the high-purity refinement method according to an embodiment of the present disclosure includes three steps.

In a first step S100, a gas is sent inside and pressurized through the first inflow line 300a connected to the first adsorption bed 100, whereby a high-adsorbent component is adsorbed and an object gas is discharged through the discharge line 400a connected to the first adsorption bed 100.

In a second step S200, a remaining object gas is sent into the second adsorption bed 200 through the collection lines 600a and 600b connected to the first adsorption bed 100 before the first adsorption bed 100 is saturated with the high-adsorbent component.

In a third step S300, the first adsorption bed 100 is vented and a supply gas is sent inside and pressurized through the collection lines 600a and 600b connected to the second adsorption bed 200.

Similarly, a gas is sent inside and pressurized through the first inflow line 300b connected to the second adsorption bed 200, whereby a high-adsorbent component is adsorbed and an object gas is discharged through the discharge line 400b connected to the second adsorption bed 200.

A remaining object gas is sent into the first adsorption bed 100 through the collection lines 600a and 600b connected to the second adsorption bed 200 before the second adsorption bed 200 is saturated with a high-adsorbent component.

The second adsorption bed 200 is vented and a supply gas is sent inside and pressurized through the collection lines 600a and 600b connected to the first adsorption bed 100.

In this way, the steps described above, that is, the first step S100 to the third step S300 are periodically switched and repeated in the first adsorption bed 100 and the second adsorption bed 200.

In particular, the dynamic pressure time that is provided when the first adsorption bed 100 and the second adsorption bed 200 are switched is separately provided to an upper portion and a lower portion.

In the high-purity refinement method according to the present disclosure, at the lower portion, the first inflow lines 300a and 300b through which a supply gas is supplied and the second inflow line 300c through which a gas is supplied to the dynamic pressure lines 500a and 500b for the dynamic pressure time are connected in parallel.

In this case, for production, the supply gas that is supplied through the first inflow lines 300a and 300b and the supply gas that is supplied through the second inflow line 300c for recycling are simultaneously supplied.

Accordingly, since a supply gas, which is the same as the supply gas that is supplied through the dynamic pressure lines 500a and 500b, is supplied to the lower portion of the first adsorption bed 100 and the lower portion of the second adsorption bed 200 through the second inflow line 300c before beds are switched, there is an effect that it is possible to prevent a possibility of damage to the adsorbent in the adsorption beds due to shock of pressure swing that rapidly changes when beds are switched.

That is, at the lower portion, the supply gas that is supplied through the dynamic pressure lines 500a and 500b to the lower portions of the first adsorption bed 100 and the second adsorption bed 200 for a dynamic pressure time before beds are switched is supplied and dynamically pressurized as a same as a the inflow gas that is supplied through the first inflow lines 300a and 300b.

Further, the high-purity refinement method according to the present disclosure includes a pressure sensor and a flow rate sensor is included in the discharge lines 400a, 400b, and 400, and may include a control system 2000 that communicates with the pressure sensor and the flow rate sensor.

The pressure sensor senses the pressure of the first adsorption bed 100 and the second adsorption bed 200 and the flow rate sensor senses the amount of gas of the first adsorption bed 100 and the second adsorption bed 200.

Further, the control system 2000 adjusts the producing time and recycling time of an object gas on the basis of predetermined conditions of the pressure sensor and the flow rate sensor, depending on the use amount of the object gas.

That is, it is possible to be always supplied with a predetermined amount of a high-purity refined gas by installing the pressure sensor and the flow rate sensor in the discharge lines 400a, 400b, and 400 and changing the producing time and the recycling time through the control system 2000, depending on the use amount of the object gas that is discharged. Further, since a high-purity gas is maintained even through the object gas is used in small amount or is not used, so stability of purity is secured. Further, it is possible to reduce energy that is consumed by the high-purity refinement system 1000 by controlling the recycling time and the producing time, depending on the use amount. Further, a gas that is the same as the collection gas is supplied to the adsorption beds when the beds are switched, so the pressure swing in the adsorption beds is small, whereby stability of the adsorbent can be achieved.

According to the present disclosure, there is an effect that it is possible to stably obtain a high-purity object gas in comparison to a pressure adsorption method of the related art by securing a process for a dynamic pressure time to improve pressure swing, which is generated in a producing and recycling process of the pressure adsorption method of the related art, and to increase production efficiency.

Various preferred embodiments of the present disclosure were described above through some examples, but the various embodiments described in "detailed description of the invention" are only examples and it would be clearly understood by those skilled in the art the present disclosure may be changed in various ways or equivalently implemented from the above description.

Further, it should be noted that since the present disclosure may be implemented in other various ways, the present disclosure is not limited to the above description, the above description is provided to completely explain the present disclosure and provided only to completely inform those skilled in the art of the range of the present disclosure, and the present disclosure is defined by only claims.

## Claims

1. A high-purity gas refinement system comprising a first adsorption bed and a second adsorption bed having an adsorbent therein, connected to a discharge line and a collection line at a first side, and connected to a first inflow line and a dynamic pressure line at a second side,
wherein a dynamic pressure time that is provided when the first adsorption bed and the second adsorption bed are switched is separately provided to an upper portion and a lower portion, and
at the lower portion, a supply gas that is supplied through the dynamic pressure lines to the lower portion of the first adsorption bed and the lower portion of the second adsorption bed for a dynamic pressure time before the beds are switched is supplied and dynamically pressurized as a gas the same as an inflow gas that is supplied through the first inflow lines.

2. The high-purity gas refinement system of claim 1, wherein, at the lower portion, a first inflow line through which a supply gas is supplied, the dynamic pressure lines, and a second inflow line connected to the dynamic pressure line are connected in parallel.

3. The high-purity gas refinement system of claim 2, wherein a supply gas that is supplied through the first inflow line and a supply gas that is supplied through the second inflow line for dynamic pressure in recycling are simultaneously supplied.

4. The high-purity gas refinement system of claim 1, comprising:
a pressure sensor and a flow rate sensor in the discharge line; and
a control system adjusting a producing time and recycling time of an object gas on the basis of predetermined conditions of the pressure sensor and the flow rate sensor, depending on a use amount of the object gas.

5. The high-purity gas refinement system of claim 2, comprising
a plurality of first check valve in the first inflow line,
wherein a gas that is supplied to the dynamic pressure lines when the beds are switched is separately provided to the first adsorption bed and the second adsorption bed through the first check valves.

6. The high-purity gas refinement system of claim 1, wherein a plurality of third check valves installed in a supply direction of a supply gas and preventing backflow of supplied gas flow and a second solenoid valve controlling the flow of a supplied gas are included in the dynamic pressure lines.

7. The high-purity gas refinement system of claim 1, wherein a plurality of second check valves installed in a collection direction of an object gas and preventing backflow of collected gas flow and a first solenoid valve controlling the flow of a collected gas are included in the collection lines.

8. The high-purity gas refinement system of claim 1, wherein a gas for recycling is collected through the collection lines for the dynamic pressure time at the upper portion.

9. A high-purity gas refinement method comprising:
a step in which a gas is sent inside and pressurized through a first inflow line connected to a first adsorption bed, whereby a high-adsorbent component is adsorbed and an object gas is discharged through a discharge line connected to the first adsorption bed;
a step in which a remaining object gas is sent into a second adsorption bed through collection lines connected to the first adsorption bed before the first adsorption bed is saturated with the high-adsorbent component; and
a step in which the first adsorption bed is vented and a supply gas is sent inside and pressurized through collection lines connected to the second adsorption bed,
wherein the steps are periodically switched and repeated in the first adsorption bed and the second adsorption bed,
a dynamic pressure time that is provided when the first adsorption bed and the second adsorption bed are switched is separately provided to an upper portion and a lower portion, and
at the lower portion, a supply gas that is supplied through the dynamic pressure lines to the lower portion of the first adsorption bed and the lower portion of the second adsorption bed for a dynamic pressure time before the beds are switched is supplied and dynamically pressurized as a gas the same as an inflow gas that is supplied through the first inflow lines.

10. The high-purity gas refinement method of claim 9, wherein, at the lower portion, a first inflow line through which a supply gas is supplied, the dynamic pressure lines, and a second inflow line connected to the dynamic pressure line are connected in parallel.
